# EUROPEAN PATENT SPECIFICATION

(11) **EP 3 551 743 B1**
(45) Date of publication and mention of the grant of the patent: **27.09.2023**
(21) Application number: 17821714.7
(22) Date of filing: 06.12.2017
(51) Int. Cl.: C12M 1/107, C12M 1/33, C12M 1/00

(54) **ADSORBENT FOR ANAEROBIC DIGESTION PROCESSES**
ADSORPTIONSMITTEL FÜR ANAEROBE VERGÄRUNGSPROZESSE
ADSORBANT POUR DES PROCÉDÉS DE DIGESTION ANAÉROBIE

(30) Priority: 06.12.2016 GB 201620753
(43) Date of publication of application: 16.10.2019
(73) Proprietor: The University Court Of The University of Edinburgh, Edinburgh, Midlothian EH8 9YL (GB)
(72) Inventor: MUMME, Jan, Edinburgh EH9 3JN (GB); SROCKE, Franziska, Edinburgh EH9 3JN (GB); FREE, Andrew, Edinburgh EH9 3JN (GB); PIETRZYK, Julian, Edinburgh EH9 3JN (GB)
(74) Representative: Murgitroyd & Company
(86) International application number: PCT/GB2017/053673
(87) International publication number: WO 2018/104730

(56) References cited:
- EP-A1- 0 025 919
- WO-A1-2015/003273
- WO-A1-2015/077484
- WO-A2-2011/018505
- WO-A2-2011/097183
- DE-A1-102011 010 525
- DE-A1-102014 100 850
- US-A1- 2016 138 048
- ZHANG MING ET AL: "Phosphate removal ability of biochar/MgAl-LDH ultra-fine composites prepared by liquid-phase deposition", CHEMOSPHERE, vol. 92, no. 8, 29 November 2013 (2013-11-29), pages 1042-1047, XP028673009, ISSN: 0045-6535, DOI: 10.1016/J.CHEMOSPHERE.2013.02.050
- PARK J H ET AL: "Evaluation of phosphorus adsorption capacity of sesame straw biochar on aqueous solution: influence of activation methods and pyrolysis temperatures", ENVIRONMENTAL GEOCHEMISTRY AND HEALTH, KEW, GB, vol. 37, no. 6, 4 June 2015 (2015-06-04), pages 969-983, XP035575609, ISSN: 0269-4042, DOI: 10.1007/S10653-015-9709-9 [retrieved on 2015-06-04] cited in the application

## Description

### Field of the Invention

The invention relates to the field of fermentation, such as anaerobic digestion, more specifically to adsorbents used in fermentation processes.

### Background of the Invention

Various types of organic and mineral adsorbents are used in anaerobic digestion for biogas production. They bind inhibitory substances like organic acids, cyanides such as those present in cassava waste, phenols such as those present in olive oil waste, ammonium and hydrogen sulphide in the fermentation liquor, and they support growth of biogas-producing microorganisms on their surfaces leading to productive biofilms. This includes the use of carbon-rich materials like charcoal, including so-called "biochar", and activated carbon, which are relatively easy to produce and don't interfere with subsequent use of the digestate e.g. as organic fertilizer or an alternative fuel. They even improve both the energetic and fertilizer value of the digestate.

DE 10 2011 010 525 discloses an adsorbent for the cleaning of biogas, which is produced from animal products like bones and part of bones. WO 2011/018505 describes a device and process for production of biogas and biochar and for refinement of the biochar. DE 10 2014 100 850 discloses the production and use of magnetisable biochar particles for biogas plants.

The main disadvantage of the current state of the art in carbon-based adsorbents for anaerobic digestion is their low surface-to-volume and surface-to-mass ratio. The surface area of the adsorbent defines its effectiveness in terms of sorption capacity and available space for biogas-producing biofilms. Porous adsorbents with a high inner surface could initially adsorb high amounts of dissolved chemicals, but over time the pores will become blocked by fine particles in the biogas slurry and microbial growth. This reduces the ability of sorption and desorption and biofilms, which grow on the inner surface, will suffer from lack of substrates. In consequence, granular adsorbents even with high inner surface area show a relative low effectiveness in biogas plants in terms of adsorption of inhibitory substances and growth of biogas-producing biofilms. The use of powdery adsorbents with a very small particle size below 10 µm will increase the sorption capacity in biogas plants, but such small particles are not large enough for biofilm formation. Further, they cannot be retained in the biogas plant and they bear the risk of dusting during storage and transportation.

Therefore, there is a need for an improved adsorbent for use in fermentation processes such as anaerobic digestion.

Phosphorus is an essential plant nutrient. Its limited accessibility has become an increasing concern for global food security. On the other hand, anthropogenic release of phosphorus is a major cause of eutrophication which is a great threat for fresh water and ecosystems. In consequence, several biological, chemical and physical methods have been developed to separate phosphorus from waste streams esp. waste water and to turn it into a fertilizer. This includes phosphate precipitation by a coagulant like calcium, aluminium and iron. Also struvite precipitation is considered which also contains nitrogen (as ammonium) and usually requires the addition of magnesium. Biological removal bases on microbial phosphorus take up by specialised microorganisms that accumulate phosphorus. However, because of high costs associated with the current methods of phosphorus removal the search for more feasible methods has led to investigations of carbon materials for phosphorus removal.

This includes activated carbon and the less costly biochar. Measured values for phosphorus adsorption on activated carbon were found at 0-10 mg g⁻¹ (Yao et al., 2011; Zheng et al., 2010; Park et al., 2015). For native biochar the highest measured value was only slightly higher of 12.4 mg g⁻¹ (Zheng et al., 2010). In order to increase the sorption capacity biochar was enriched with iron (Chen et al. 2011; Ren et al., 2015; Yao et al., 2011) and magnesium (Zhang et al., 2012; Zhang et al. 2013; Yao et al., 2013a) have been investigated. The highest sorption capacity was shown by biochar/MgAI-layered double hydroxides ultra-fine composites prepared by liquid-phase deposition (Zhang et al., 2013). This material was found with a calculated sorption capacity for phosphorus of 134 mg g⁻¹ (PO₄ = 410 mg g⁻¹).

However, these techniques are very complex and costly, and therefore, there is a need for effective adsorbents for phosphorus at much lower costs.

Accordingly, it is at least one object of the invention to provide an improved adsorbent.

Carbon adsorbents known in the art are typically made up of particulate carbon materials like activated carbon or biochar, and are used for various purposes e.g. filtering and separation, catalysis, soil amendment, energy storage, and carbon sequestration. Several of these applications require or prefer agglomerated carbon particles instead of loose particles. Other applications (e.g., composting, stabilization and deodorisation of sewage sludge and agricultural wastes, biogas production, and soil amendment), however, require a dispersion of fine carbon particles in the treated medium for full effectiveness. These applications are characterized by a large media volume where the carbon particles are expected to distribute as evenly and energy efficiently as possible.

Nevertheless, handling these materials before their targeted use is considerably more convenient and economically efficient in agglomerated instead of a loose form. This includes packaging, transportation, storage and dosage as these agglomerates require less volume, are less likely to dust and are less prone to wetting. Furthermore, the size, density and robustness of the agglomerates can be designed to meet application-specific requirements. In consequence, for these applications the ideal solutions are agglomerated carbon particles that disintegrate rapidly when put into use.

These applications usually require the carbon particles to work in a water-rich environment, and as a result a water soluble type of binder can be used to achieve disintegration. Soluble binders such as sugars or several types of silicates have been used for several decades for agglomeration e.g. of pharmaceutical products. For agglomeration of carbon particles, however, there is limited information available on the use of soluble binders. Published information that is available include results from wet-drum granulation of biochar using the soluble binder hydroxypropyl methylcellulose (HPMC), molasses and ammonium nitrate (Bowden- Green and Briens, 2016; Bowden-Green 2016). However, none of the produced granules were found to be sufficiently stable according to industrial standards.

WO2015/077484 A1 discloses a particulate carbon adsorbent as an additive for an bioreactor.

Accordingly, there is a need for improved granules of carbon adsorbents.

Therefore, it is at least one object of the invention to provide improved adsorbent for use in fermentation processes such as anaerobic digestion.

### Summary of the Invention

According to a first aspect of the invention there is provided the use of a particulate carbon adsorbent in anaerobic digestion, the particulate carbon adsorbent being substantially planar and comprising between 40-90 wt% carbon, characterised in the particulate carbon adsorbent is obtainable by pyrolysing a substantially planar organic material at a temperature from 300°C to 800°C, grinding the pyrolysed material; and sorting the ground pyrolysed material to specify the dimensions of the particulate carbon adsorbent.

The particulate carbon adsorbent is formed by pyrolysis of substantially planar organic material. Pyrolysis is a thermochemical decomposition of organic materials at elevated temperatures in the absence of oxygen, or in low levels or reduced levels of oxygen.

By the term "substantially planar" we refer to a material that is formed of at least one sheet, wherein the length and width of the sheet extends by at least 5 times the thickness of the sheet. Accordingly, the particulate carbon adsorbent may be considered to be carbon flakes. The at least one sheet may be curved, folded, creased or otherwise deformed from the original plane of the sheet, but retains on at least a local scale the properties of the thickness of the material being at least 5 times less the extent of the material in the remaining two dimensions. For example, a sheet of paper is included in the definition of a substantially planar material whether that sheet of material is folded, creased or otherwise deformed form the original plane of the sheet of paper.

The inventors have found that substantially planar carbon adsorbents are particularly effective for promoting the production of biogas or biofuel in anaerobic digestion. The substantially planar geometry of the particulate carbon adsorbents or carbon flakes have been found to both effectively adsorb inhibitory species from the aqueous solution typically found within bioreactors that are produced as a by-product of the anaerobic digestion process, and to provide an ideal substrate for the formation of a productive biofilm of microorganisms that are typically used in anaerobic digestion in bioreactors. In one example, the particulate carbon adsorbents may be suitable substrates for the formation of biofilms formed by methanogenic bacteria and/or archaea.

The anaerobic digestion may be carried out in a bioreactor, and as a result, the particulate carbon adsorbent may be used in a bioreactor. The bioreactor may be used to produce biofuel by anaerobic digestion of a feedstock by a microorganism or a consortium of different microorganisms, and the particulate carbon adsorbent may adsorb at least some of the by-products of anaerobic digestion that may inhibit further production of biofuel. The inhibitory by-products of anaerobic digestion may include organic acids, including C1-C10 organic acids, ammonia, and hydrogen sulphide. The organic acids may include C1-C6 organic acids. The organic acids may include propionic acid (C3 organic acid).

In embodiments, the particulate carbon adsorbent may be added to the feedstock of the bioreactor as a powder or in a granular form prior to initiation of anaerobic digestion. The particulate carbon adsorbent may be added regularly to the feedstock of the bioreactor as a powder or in a granular form with a batching process of anaerobic digestion. The particulate carbon adsorbent may be added continuously to the feedstock of the bioreactor as a powder or in a granular form in a continuous process of anaerobic digestion. The particulate carbon adsorbent may be dispersed throughout the feedstock of the bioreactor to ensure that the particulate carbon adsorbent is able to efficiently adsorb inhibitory by-products and therefore enhance the production of biogas or biofuel in the bioreactor.

The particulate carbon adsorbent may provide a substrate for the formation of an active biofilm. The substantially planar geometry of the particulate carbon adsorbent provides a larger outer surface area upon which the microorganisms can colonise and produce an active biofilm than known absorbents with similar volume. The surface of the particulate carbon adsorbent may provide sufficient porosity to provide protected or sheltered environments within which microorganisms may thrive, whilst at the same time having sufficiently shallow pores so that the microorganisms that colonise the pores have ready access to the nutrients they require to thrive.

In some embodiments, the particulate carbon adsorbent may comprise a pre-formed biofilm prior to use. Accordingly, the particulate carbon adsorbent may be pre-inoculated with suitable microorganisms to allow a ready-to-activate biofilm to form on the surface of the particulate carbon adsorbent.

The particulate carbon adsorbent may be pre-inoculated with microorganisms within fluid obtained from the target system at any stage of the anaerobic digestion process. Accordingly, the biofilm formed on the particulate carbon adsorbent may comprise microorganisms that are naturally found in the target system, thereby ensuring that the particulate carbon adsorbent already has a potentially productive biofilm before being added to the target system.

For example, in embodiments where the anaerobic digestion occurs in a bioreactor, the particulate carbon adsorbent may be pre-inoculated with fluid obtained from the bioreactor at any stage of the anaerobic digestion process.

In another example, the particulate carbon adsorbent may be pre-inoculated with fluid obtained from a bioreactor that processes the same or similar feedstock as the target system at any stage of the anaerobic digestion process.

In a yet further example, the particulate carbon adsorbent may be pre-inoculated with fluid that comprises a mix of microorganisms that are active in anaerobic digestion processes and/or are beneficial in anaerobic digestion bioreactors. The fluid may be specifically tailored such that the fluid comprises specific identified microorganisms that are beneficial for anaerobic digestion. The specific microorganisms may have been isolated from a natural source, or may have been cultured artificially.

The substantially planar organic material may comprise paper or cardboard or similar. The substantially planar organic material may comprise leaves from plants, or other substantially planar organic materials. The substantially planar organic material may comprise a plurality of layers of substantially planar organic material.

The pyrolysis of substantially planar organic material has the effect that the particulate carbon adsorbent formed therefrom is also substantially planar. Accordingly, the form and shape of the substantially planar organic material may be transferred to the particulate carbon adsorbent formed therefrom.

In some embodiments, the substantially planar organic material may be a composite material. The composite material may be formed from layers of paper and layers of plastic or wax. The substantially planar organic material may be formed from reinforced paper, which comprises at least one layer of paper and at least one layer of plastic or wax. The at least one layer of plastic or wax may be a coating of plastic or wax on the at least one layer of paper.

In embodiments where the substantially planar organic material comprises paper, the paper typically comprises cellulose fibres derived from wood, cloth or grasses. Accordingly, the particulate carbon adsorbent may comprise fibre formations, and these formations may provide suitable colony sites for microorganisms such as bacteria and archaea used in anaerobic digestion.

The substantially planar organic material may comprise waste material. The substantially planar organic material may comprise waste paper. In embodiments where the substantially planar organic material comprises at least one layer of paper and at least one layer of plastic or wax, the substantially planar waste material may comprise waste paper cups.

In embodiments where the particulate carbon adsorbent comprises an active biofilm, during use the particulate carbon adsorbent may comprise bubbles of gases such as methane, hydrogen or carbon dioxide or mixtures thereof that are trapped at the surface. Accordingly, these particulate carbon adsorbents may become at least neutrally buoyant and thereby resist sedimentation. Therefore, in embodiments where the anaerobic digestion process is occurring within a bioreactor, the particulate carbon adsorbent may remain suspended within the liquid within the bioreactor during use. The particulate carbon adsorbents may become positively buoyant and thereby float to the surface of the liquid within the bioreactor during use. Accordingly, at a significant proportion of the particulate carbon adsorbents may be retained within the bioreactor when the digestate is removed from the bottom of the bioreactor, for example.

The particulate carbon adsorbent may comprise calcium and/or magnesium ions. Particulate carbon adsorbents comprising calcium and/or magnesium ions may be better substrate for the microbial colonisation. Accordingly, particulate carbon adsorbents that comprise calcium and/or magnesium ions may more readily form microbial biofilms on the surface of the particulate carbon adsorbents than particulate carbon adsorbents that do not comprise calcium and/or magnesium ions. Microbial biofilms formed on the surface of particulate carbon adsorbents may be more viable or more productive where the particulate carbon adsorbent comprises calcium and/or magnesium ions.

Calcium or magnesium ions may be added to the particulate carbon adsorbent after the particulate carbon adsorbent has been formed. The substantially planar organic material may comprise calcium and/or magnesium salts and those salts may at least partially survive the pyrolysis process
In embodiments where the particulate carbon adsorbent is formed from pyrolysis of paper, the calcium or magnesium may be component of the filler of the paper. For example, the paper may comprise a filler that comprises CaCO₃ (calcium carbonate), MgCO₃ (magnesium carbonate), MgO (magnesium oxide), Mg(OH)₂ (magnesium hydroxide), CaMg(CO₃)₂ (dolomite) or talc (which comprises magnesium and typically has the chemical formula H₂Mg₃(SiO₃)₄ or Mg₃Si₄O₁₀(OH)₂).

The particulate carbon adsorbent may comprise from about 0.1 wt % to about 10 wt% calcium ions. The particulate carbon adsorbent may comprise from about 1.0 wt% to about 15.0 wt% magnesium ions.

The particulate carbon adsorbent may comprise both calcium ions and magnesium ions.

The particulate carbon adsorbent may comprise phosphorus. The particulate carbon adsorbent may comprise nitrogen or a nitrogen oxide.

In some embodiments, the particulate carbon adsorbent has a major dimension and extends at least 0.05mm along the major dimension. The particulate carbon adsorbent may extend at least 1.0mm along the major dimension.

Typically, the particulate carbon adsorbent does not extend more than 5mm along the major dimension.

For example, the particulate carbon adsorbent may extend along the major dimension from 0.05mm to 5mm. The particulate carbon adsorbent may extend along the major dimension from 0.1mm to 1.2mm. The particulate carbon adsorbent may extend along the major dimension from 0.1mm to 1.0mm. The particulate carbon adsorbent may extend along the major dimension from 0.1 mm to 0.5mm.

The particulate carbon adsorbent may have a thickness of less than 0.3mm. The particulate carbon adsorbent may have a thickness of less than 0.2mm. The particulate carbon adsorbent may have a thickness of less than 0.1mm. The particulate carbon adsorbent may have a thickness of less than 0.05mm. The particulate carbon adsorbent may have a thickness of less than 0.02mm. The particulate carbon adsorbent may have a thickness of less than 0.01mm.

Typically, the particulate carbon adsorbent is in the form of a granular material. The carbon particles of the particulate carbon adsorbent within the granular material are substantially planar or "flake-shaped". The particulate carbon adsorbent granular material may be added to an aqueous solution comprising the feedstock for the anaerobic digestion such that the particulate carbon adsorbent is dispersed throughout the aqueous solution.

The particulate carbon adsorbent may comprise an aqueous composition, and the aqueous composition may comprise one or more compounds that promote biofilm formation. For example, the aqueous composition may comprise a buffering agent that maintains the pH of the aqueous composition to pH 6 to pH 8. The aqueous composition may comprise macro- and/or micro-nutrients for the microorganisms that are to colonise the surface of the particulate carbon adsorbent to form the biofilm.

The aqueous composition may comprise phosphorus containing compounds or salts. The aqueous composition may comprise nitrogen containing compounds or salts. The aqueous composition may comprise magnesium containing compounds or salts. The aqueous composition may comprise calcium containing compounds or salts.

In some embodiments, the concentration of the particulate carbon adsorbent to the feedstock may be about 0.5-3.0 wt%. Typically, the concentration of the particulate carbon adsorbent added to the feedstock will be dependent on the type and amount of feedstock used in the anaerobic digestion process. For example, in embodiments where the reactor liquid is more viscous (from high solid feedstocks such as food waste, maize silage or solid manure, for example), the particulate carbon adsorbent may be required to be replaced at a higher rate than embodiments where the reactor liquid is less viscous. In another example, in embodiments where there is a higher risk and/or a higher severity of inhibition of the anaerobic digestion process, the higher the required concentration of flakes or particulate carbon adsorbent. A higher risk of inhibition or a higher severity of inhibition may occur with higher concentrations of inhibitors in the feedstock materials or their precursors (e.g. organic nitrogen to ammonia), with higher degradation rates (e.g. due readily available feedstock and higher process temperatures), short retention time (e.g. due to higher water content of the feedstock), low buffer capacity (e.g. water rich feedstocks), and high fluctuations in digester feeding (e.g. type and dosage of feedstock), for example.

The particulate carbon adsorbent may comprise a magnetic species. The magnetic species may allow the particulate carbon adsorbent used in the anaerobic digestion process to be readily separated from the digestate to allow the particulate carbon adsorbent to be reused in further anaerobic digestion processes. The magnetic species may be ferrites.

The invention extends in a second aspect to a method of manufacture of particulate carbon adsorbents for the use of the first aspect, the method comprising the steps:
a) providing a substantially planar organic material;
b) pyrolysing the provided substantially planar organic material at a temperature from 300°C to 800°C;
c) grinding the pyrolysed material resulting from step b); and
d) sorting the ground pyrolysed material resulting from step c) to specify the dimensions of the particulate carbon adsorbent required.

The substantially planar organic feedstock may be pyrolysed at a temperature from 300°C to 500°C.

The substantially planar organic feedstock may be pyrolysed at a temperature from 350°C to 500°C. Preferably, the substantially planar organic feedstock is pyrolysed at a temperature from 400°C to about 475°C. More preferably, the substantially planar organic feedstock is pyrolysed at a temperature of about 450°C.

The substantially planar organic feedstock may be pyrolysed at a temperature from 400°C to about 475°C if high yields and low alkalinity of the resulting pyrolysed material are required. The substantially planar organic feedstock may be pyrolysed at a temperature from 650°C to about 800°C if high alkalinity, high porosity and high electrical conductivity of the resulting pyrolysed material are required. The substantially planar organic feedstock may be pyrolysed at a temperature of about either 450°C or 750°C depending on the desired properties of the resulting pyrolysed material.

In some embodiments, the substantially planar organic material may be shredded before it is pyrolysed.

The shredded substantially planar organic material may be compressed before the step of pyrolysing the substantially planar organic material. The shredded substantially planar organic material may be compressed into a block. For example, the shredded substantially planar organic material may be compressed into a block of about 3cm, 4cm or 5cm in diameter. The block may be a compressed briquette or pellet form of the shredded substantially planar organic material.

Typically, the substantially planar organic material is paper or cardboard. In some embodiments, the substantially planar organic material may include laminated paper comprising at least one layer of paper and at least one layer of plastic or wax. The substantially planar organic material may include laminated paper comprising at least one layer of paper and one layer of aluminium. For example, the substantially planar organic material may comprise multiple layers of paper and a single layer of a plastic such as polyethylene or polylactic acid (PLA) or single layer of wax. In embodiments where the substantially planar organic material comprises paper cups or cartons, the layer of plastic or wax may cover the inner surface of the cup or carton to ensure that the food or beverage product may be retained within the cup or carton without absorption of the moisture into the paper layers leading to degradation of the integrity of the cup or carton. The substantially planar organic material may comprise at least two layers of a plastic or wax. In embodiments where the substantially planar organic material comprises paper cups or cartons, the paper cup or cartons may comprise a plastic or wax layer on the inner surface and a plastic or wax layer on the outer surface.

In embodiments where the substantially planar organic material is paper, the paper may comprise a filler, and the filler may comprise calcium and/or magnesium.

The substantially planar particulate carbon adsorbents may be mixed and then incubated with fluid from a target system within which the substantially planar particulate carbon adsorbents are intended to be used. The substantially planar particulate carbon adsorbents may be incubated with the fluid for at least 12 hours, at least 24 hours or at least 36 hours. Incubation may allow microorganisms to adhere and a biofilm to form on the substantially planar particulate carbon adsorbents. The biofilm may comprise microorganisms from the target system. Accordingly, the biofilm of the substantially planar particulate carbon adsorbents may comprise microorganisms that are beneficial to anaerobic digestion.

The step of sorting the ground pyrolysed material may be carried out by sieving or sifting the material to isolate particulates that fall within a chosen particle range.

According to a third aspect of the invention, there is provided an anaerobic digestion process comprising the steps:
a) providing a bioreactor;
b) adding a feedstock to the bioreactor;
c) adding a microorganism composition to the bioreactor;
d) adding an adsorbent composition to the bioreactor, the adsorbent composition comprising substantially planar particulate carbon adsorbent as defined herein;
e) incubating the bioreactor such that microorganisms within the microorganism composition digest the feedstock to produce a digestate and biogas; and
f) removing the produced biogas and digestate.

Typically, the feedstock comprises organic waste, such as waste from sewage plants, food and beverage waste, processing residues such as bakery and brewery waste, agricultural residues such as straw, leaves, or unwanted fruit and vegetables, and specifically grown crops such as maize, grass silage, energy beet and wholecrop cereals.

The microorganism composition may comprise bacteria that hydrolyse the feedstock. The microorganism composition may comprise acidogenic bacteria that convert sugars and amino acids into carbon dioxide, hydrogen, ammonia and organic acids. Bacteria within the microorganism composition may convert organic acids into acetic acid, formic acid, hydrogen and carbon dioxide. The microorganism composition may comprise methanogenic archaea that are able to convert the products of feedstock hydrolysis into methane and carbon dioxide. Typically, the microorganism composition comprises a combination of types of bacteria and archaea such that the microorganism composition is capable of converting the feedstock added to the bioreactor into biogas and digestate.

Carbon dioxide and methane produced by the microorganism composition forms the biogas that is typically the desired product of the bioreactor.

Typically, the substantially planar particulate carbon adsorbent adsorbs soluble compounds of the feedstock and by-products of the anaerobic digestion process. Typically, the substantially planar particulate carbon adsorbent adsorbs soluble compounds of the feedstock and by-products of the anaerobic digestion process that inhibit the anaerobic digestion process either already at the beginning or during the production process. The inhibitory compounds of the feedstock or by-products of anaerobic digestion may include organic acids, including C1-C10 organic acids, ammonia, and hydrogen sulphide. Further, free ammonia (NH₃) and hydrogen sulphide (H₂S) in addition to their inhibitory potential also decrease the biogas quality, thus they need to be removed before the biogas can be used as fuel. Binding them on the substantially planar particulate carbon adsorbent will reduce their concentration in the biogas and thus reduce the need for external biogas cleaning. Typically, the substantially planar particulate carbon adsorbent also adsorb CO₂ which increases the methane content of the produced biogas and thereby it increases the biogas' energetic value and simplifies upgrading of biogas into bio-methane.

Also disclosed is the use of a particulate carbon adsorbent in phosphorous capture, the particulate carbon adsorbent being substantially planar and comprising between 40-90 wt% carbon, and comprising between 1-15wt% magnesium ions. The particulate carbon adsorbent may comprise from 1-15wt% magnesium ions. Preferably at least 30% of the magnesium is MgO (magnesium oxide). Table 1 shows the variations in the total content of calcium and magnesium; and the distribution of magnesium bonding types. Because the MgO (magnesium oxide) is the dominant binder of phosphorous, pyrolysis temperatures above 450°C are preferred for phosphorous binding over pyrolysis temperatures below 450°C.

**Table 1: Total content of Ca and Mg and ratios of Mg bonding types in particulate carbon adsorbents (NA = not available) prepared from paper cups.**

| Substantially planar organic material | Pyrolysis temperature | Ca | Mg | Mg bonding types identified by XRD (% of total Mg) | | | | |
|---|---|---|---|---|---|---|---|---|
| | °C | mg/g | mg/g | Calcite | Dolomite | Talc | MgCO₃ | MgO |
| PE paper cup #1 (PC1) | 750 | 18.6 | 103.6 | 7.6 | 3.7 | 17.1 | 0.0 | 71.7 |
| | 550 | NA | NA | 0 | 0.9 | 42.6 | 25.3 | 31.3 |
| | 450 | 6.9 | 54.2 | 0 | 5.5 | 38.8 | 54.7 | 1.0 |
| PE paper cup #2 (PC2) | 750 | 76.3 | 71.6 | NA | NA | NA | NA | NA |
| | Untreated raw material | 25.1 | 13.9 | NA | NA | NA | NA | NA |
| PLE paper cup (PC3) | 750 | 19.8 | 96.1 | NA | NA | NA | NA | NA |

Typically, the particulate carbon adsorbent is formed by pyrolysis of substantially planar organic material. Pyrolysis is a thermochemical decomposition of organic materials at elevated temperatures in the absence of oxygen, or in low levels or reduced levels of oxygen.

The term "substantially planar" is defined in accordance with the first aspect.

The particulate carbon adsorbent is added to waste water to capture phosphorous (in the form of phosphate) that may be present within the waste water.

Typically, the magnesium ions within the particulate carbon adsorbent is in the form of magnesium oxide (MgO). The inventors have found that the particulate carbon adsorbent as described in the first aspect is particularly effective at adsorbing phosphorous from an aqueous solution when the particulate carbon adsorbent comprises magnesium oxide.

In embodiments where the particulate carbon adsorbent comprises magnesium oxide, the particulate carbon adsorbent may comprise from about 1 wt% to about 15 wt% of magnesium that is bound as magnesium oxide.

The substantially planar organic material may comprise paper or cardboard or similar. The pyrolysis of substantially planar organic material has the effect that the particulate carbon adsorbent formed therefrom is also substantially planar. Accordingly, the form and shape of the substantially planar organic material may be transferred to the particulate carbon adsorbent formed therefrom.

In some embodiments, the substantially planar organic material may be a composite material. The composite material may be formed from layers of paper and layers of plastic or wax. The substantially planar organic material may be formed from reinforced paper, which comprises at least one layer of paper and at least one layer of plastic or wax. The at least one layer of plastic or wax may be a coating of plastic or wax on the at least one layer of paper.

The substantially planar organic material may comprise waste material. The substantially planar organic material may comprise waste paper. In embodiments where the substantially planar organic material comprises at least one layer of paper and at least one layer of plastic or wax, the substantially planar organic material may comprise waste paper cups.

In embodiments where the particulate carbon adsorbent is formed from pyrolysis of paper, the magnesium may be component of the filler of the paper. For example, the paper may comprise a filler that comprises CaCO₃ (calcium carbonate), MgCO₃ (magnesium carbonate), MgO (magnesium oxide), Mg(OH)₂ (magnesium hydroxide), CaMg(CO₃)₂ (dolomite) or talc (which comprises magnesium and typically has the chemical formula H₂Mg₃(SiO₃)₄ or Mg₃Si₄O₁₀(OH)₂).

In some embodiments, the particulate carbon adsorbent has a major dimension and extends at least 0.05mm along the major dimension. The particulate carbon adsorbent may extend at least 1.0mm along the major dimension.

Typically, the particulate carbon adsorbent does not extend more than 5mm along the major dimension.

For example, the particulate carbon adsorbent may extend along the major dimension from 0.05mm to 5mm. The particulate carbon adsorbent may extend along the major dimension from 0.1mm to 1.2mm. The particulate carbon adsorbent may extend along the major dimension from 0.1mm to 1.0mm. The particulate carbon adsorbent may extend along the major dimension from 0.1 mm to 0.5mm.

The particulate carbon adsorbent may have a thickness of less than 0.3mm. The particulate carbon adsorbent may have a thickness of less than 0.2mm. The particulate carbon adsorbent may have a thickness of less than 0.1mm. The particulate carbon adsorbent may have a thickness of less than 0.05mm. The particulate carbon adsorbent may have a thickness of less than 0.02mm. The particulate carbon adsorbent may have a thickness of less than 0.01mm.

Typically, the particulate carbon adsorbent is in the form of a granular material. The carbon particles of the particulate carbon adsorbent within the granular material are substantially planar or "flake-shaped".

During use, the particulate carbon adsorbent may be removed from the waste water once the phosphorous (or phosphate) within the waste water has been captured by the particulate carbon adsorbent. For example, the waste water may be filtered. In another example, the particulate carbon adsorbent may tend to float to the surface of the waste water and the particulate carbon adsorbent may be skimmed from the surface of the waste water. Further, the particulate carbon adsorbent may settle and then be removed from the waste water. Settling can occur naturally or enforced by using a centrifuge.

After use, the particulate carbon adsorbent is typically enriched with phosphorous. The enriched particulate carbon adsorbent may be used to amend soil to provide an additional source of phosphorous. Accordingly, the enriched particulate carbon adsorbent may be used as a fertiliser, or added to enrich a fertiliser. Further, the enriched particulate carbon adsorbent can also be used as additive in anaerobic digestion plants. For example, the particulate carbon adsorbent can be added to the aerobic treatment step of a waste water treatment plant to adsorb phosphate. The enriched particulate carbon adsorbent can then be transferred into an anaerobic digester together with the sewage sludge that is produced in the same waste water treatment plant.

Also disclosed is a method of manufacture of particulate carbon adsorbents for the use of use of a particulate carbon adsorbent in phosphorous capture, the particulate carbon adsorbent being substantially planar and comprising between 40-90 wt% carbon, and comprising between 1-15wt% magnesium ions, the method comprising the steps:
a) providing a substantially planar organic material;
b) pyrolysing the provided substantially organic material at a temperature from 550°C to 800°C;
c) grinding the pyrolysed material resulting from step b); and
d) sorting the ground pyrolysed material resulting from step c) to specify the dimensions of the particulate carbon adsorbent required.

The substantially planar organic material may be pyrolysed at a temperature from 550°C to 800°C, while 600°C to 750°C are preferred. During the pyrolysis process, the pyrolysis reactor may be purged with an inert gas e.g. N₂, in order to foster the formation of MgO by removing CO₂ and other gases from the reactor.

In some embodiments, the substantially planar organic material may be shredded before it is pyrolysed.

The shredded substantially planar organic material may be compressed into briquettes or pellets before the step of pyrolysing the substantially planar organic material.

The shredded substantially planar organic material may be compressed before the step of pyrolysing the substantially planar organic material. The shredded substantially planar organic material may be compressed into a block. For example, the shredded substantially planar organic material may be compressed into a block (briquette) of about 3cm, 4cm or 5cm in diameter.

Typically, the substantially planar organic material is paper or cardboard. In some embodiments, the substantially planar organic material may include laminated paper comprising at least one layer of paper and at least one layer of plastic or bioplastic (e.g. polylactic acid (PLA)) or wax. Bioplastic is understood to be biodegradable plastic derived from biological substances rather than petroleum. For example, the substantially planar organic material may comprise multiple layers of paper and a single layer of a plastic such as polyethylene or a layer of wax. In embodiments where the substantially planar organic material comprises paper cups or cartons, the layer of plastic or wax may cover the inner surface of the cup or carton to ensure that the food or beverage product may be retained within the cup or carton without absorption of the moisture into the paper layers leading to degradation of the integrity of the cup or carton. The substantially planar organic material may comprise at least two layers of a plastic or wax. In embodiments where the substantially planar organic material comprises paper cups or cartons, the paper cup or cartons may comprise a plastic or wax layer on the inner surface and a plastic or wax layer on the outer surface.

Preferably, the substantially planar organic material comprises magnesium. For example, in embodiments where the substantially planar organic material is paper, the paper may comprise a filler that comprises CaCO₃ (calcium carbonate), MgCO₃ (magnesium carbonate), MgO (magnesium oxide), Mg(OH)₂ (magnesium hydroxide), CaMg(CO₃)₂ (dolomite) or talc which comprises magnesium and typically has the chemical formula H₂Mg₃(SiO₃)₄ or Mg₃Si₄O₁₀(OH)₂.

The step of sorting the ground pyrolysed material may be carried out by sieving or sifting the material to isolate particulates that fall within a chosen particle range.

Also disclosed is a carbon granule comprising substantially planar particulate carbon adsorbent and a binder.

The carbon granule may comprise an outer shell surrounding a core. The outer shell may comprise substantially planar particulate carbon adsorbent. The core may comprise carbon particles. The core may comprise substantially planar particulate carbon adsorbent. The core may comprise both carbon particles and substantially planar particulate carbon adsorbent.

The term "substantially planar" is defined above with respect to the first aspect, and this definition is included in the present discussion.

By the term "carbon particles" we refer to particles of carbon that are not substantially planar. The carbon particles may comprise activated carbon. Activated carbon is a form of carbon that has been processed to have a high degree of microporosity to thereby increase the surface area of the carbon.

The substantially planar particulate carbon adsorbent may be carbon flakes. The particulate carbon adsorbent within the carbon granule may be configured to adsorb species. For example, in aqueous solution, the substantially planar particulate carbon adsorbent may adsorb species from the aqueous solution. The substantially planar particulate carbon adsorbent may be particularly effective at adsorbing species from aqueous solution.

Species in the sense of the present invention refer to inhibitory species from the aqueous solution typically found within bioreactors that are produced as by-products of the anaerobic digestion process. The inhibitory by-products of anaerobic digestion may include organic acids, including C1-C10 organic acids, ammonia, and hydrogen sulphide.

The binder may be a composition that binds at least some of the particles together. For example, in embodiments where the carbon granule comprises a core and an outer shell, the binder may bind the particles within the outer shell and in the core together. Alternatively, the binder may bind the particles in the outer shell only.

The binder may be a composition that binds the particulate carbon adsorbent together and dissolves when brought into contact with water. Accordingly, the binder is typically a water soluble composition. As a result, the carbon granules may break up and disperse the particulate carbon adsorbent and carbon particles within the granules through an aqueous medium when the carbon granules are brought into contact with that aqueous medium.

The binder may be a sugar or a composition comprising a sugar. For example, the binder may be molasses. The binder may be a composition extracted from a plant source. For example, the binder may be a seaweed extract. The binder may be carboxymethyl cellulose (CMC), hydroxypropylmethyl cellulose (MPMC), ammonium nitrate or polyethylene glycol (PEG).

Alternatively, the binder may be a silicate. For example, the binder may be sodium silicate or an organic ammonium silicate.

The binder may be a composition that binds the particulate carbon adsorbent and/or carbon particles together and dissolves when brought into contact with a non-polar solvent. Accordingly, the binder is typically an oil soluble composition. As a result, the carbon granules may break up and disperse the particulate carbon adsorbent and/or carbon particles within the granules through non-polar medium when the carbon granules are brought into contact with that non-polar medium.

The binder may be an oil-based binder. The binder may be palm oil, cocoa butter, or a paraffin. The binder may be tar, lignin, lignin derivatives such as lignin sulfonate, and bentonite, such as sodium bentonite or calcium bentonite.

The carbon granules of the invention have been found to be more stable than those known in the art. The carbon granules may retain their shape when subjected to vibration or other physical stress to a greater degree than carbon agglomerates known in the art. The carbon granules may be more resistant to dusting than carbon agglomerates known in the art. As a result, more of the substantially planar particulate carbon adsorbent retain their shape and form when stored in the carbon granules of the invention than stored in other forms.

The carbon granules may be used in anaerobic digestion processes, such as those described in the third aspect of the invention. The carbon granules may be used in contaminant capture processes, such as phosphorous capture, for example.

The carbon granules may be used in waste water treatment. The carbon granules may be added to waste water to adsorb contaminants within the waste water. Once the waste water has been treated, the substantially planar particulate carbon adsorbent and/or carbon particles from the granules may be removed from the treated waste water.

The carbon granules may be used in processes for recovering materials from waste water. The carbon granules may be used to adsorb materials from waste water that may be used in other applications. For example, the substantially planar particulate carbon adsorbent within the carbon granules may be used to adsorb phosphorus and nitrogen from waste water. The enriched substantially planar particulate carbon adsorbent may then be extracted from the waste water and used to enrich the phosphorus and nitrogen content of soil or to supply phosphorous and nitrogen as a nutrient to bioprocesses such as anaerobic digestion. For example, the substantially planar particulate carbon adsorbent may adsorb phosphorus and nitrogen and be used to enrich soil.

The carbon granules may be used in water cleaning. For example, the carbon granules may be applied to water and the dispersed substantially planar particulate carbon adsorbent may adsorb contaminants within the water. The substantially planar particulate carbon adsorbent may then be extracted, resulting in cleaner water.

The carbon granules may be used in nitrification processes in waste water cleaning plants.

The carbon granules may be used in composting. The carbon granules may be used in soil amendment. The carbon granules may be applied to soil such that the water within the soil dissolves the binder and thereby releases the substantially planar particulate carbon adsorbent within the granule into the soil. Typically, the substantially planar particulate carbon adsorbent will disperse through the soil at a slower rate than carbon particles that are not substantially planar within the core of the granules. As a result, the distribution of carbon particles will vary as a function of depth through the soil, with the concentration of substantially planar particulate carbon adsorbent being greater near the surface of the soil to which the granules are applied, and the concentration of carbon particles that are not substantially planar being more uniform throughout the soil.

Accordingly, the carbon granules of the invention may be used to increase the carbon content of the soil, thereby sequestering carbon in the soil.

The carbon granules may be pre-loaded with a composition that comprises nutrients for plants that may be planted within the soil to be treated. Therefore, the carbon granules may be used to increase the nutrients within the soil to increase the growth of plants planted in the treated soil, for example.

The carbon granules may be used as a template to grow microbial biofilms comprising specific useful microorganisms. The carbon granules comprising the microbial biofilm may be used to apply the microorganisms to treat or digest a specific material. For example, the carbon granules could be used as a substrate for *Colwellia* (consume ethane), *Cycloclasticus* (consume aromatic compounds), *Oceanospirillales* (consume alkanes), *Alcanovorax* (consume the hydrocarbons that make up raw oil), or *Methylococcaceae* (consume methane), for example, and the carbon granules used to treat oil spills.

The carbon granules may comprise a biofilm. The substantially planar particulate carbon adsorbent may comprise a biofilm.

Also disclosed is a method of manufacturing the carbon granules according to the carbon granule comprising substantially planar particulate carbon adsorbent and a binder, the method comprising the steps:
a) providing a first composition comprising particulate carbon material;
b) forming a core comprising the first composition;
c) providing a second composition comprising substantially planar particulate carbon adsorbent and a binder; and
d) forming an outer shell of the second composition around the core to form a granule.

The particulate carbon material may comprise substantially planar particulate carbon adsorbent and/or carbon particles. The first composition may comprise activated carbon. The first composition may comprise substantially planar particulate carbon adsorbent. The first composition may comprise both activated carbon and substantially planar particulate carbon adsorbent.

The first composition may comprise a binder. The binder of the first composition may be the same as the binder of the second composition. The binder of the first composition may be different from the binder of the second composition.

### Brief Description of the Figures

Embodiments of the present invention will now be described, by way of non-limiting example, with reference to the accompanying drawings.
Figure 1: Scanned electron Microscopy (SEM) image of the surface of a particulate carbon adsorbent made from paper cup waste;
Figure 2: SEM image of the surface of a particulate carbon adsorbent made from paper cup waste;
Figure 3: Optical image of an example particulate carbon adsorbent;
Figure 4: Energy-dispersive spectroscopy (EDS) spectra (A and B) of elements present on different points of the surface of a particulate carbon adsorbent;
Figure 5: Course of biogas production of syringe fermenters in the first experiment with and without the addition of adsorber (particulate carbon adsorbent). Feeding of propionic acid on day 5. Diagram shows average values of 4 repetitions.
Figure 6: Course of biogas production of syringe fermenters in the first experiment with and without the addition of adsorber (particulate carbon adsorbent). Feeding of propionic acid marked by diamonds. Diagram shows average values of at least 2 repetitions.
Figure 7: A carbon granule comprising a core of smaller carbon particles (1) and a shell of larger flake-shaped particles (particulate carbon adsorbent)(2).
Figure 8: Course of the biogas production in the second anaerobic digestion experiment. The diagram shows average values of three repetitions.
Figure 9. Biogas generation from test AD reactors with the addition of 5 g/l Chemical Oxygen Demand (COD) bio-oil from softwood pellets (SWP/BO). The 20 mL of digestate was supplemented with Sodium 2-bromoethanesulfonate (BES) (Be), standard biochar (St), pre-incubated standard biochar (Si), carbon particulate adsorbent according to the invention (CreChar^{®}) (Cr) and pre-incubated CreChar^{®} (Ci). Control reactors were not supplemented (-). All conditions were performed in duplicate with each curve as a mean of the replicates with error bars of standard deviation.

### Detailed Description

### Use of Particulate Carbon Adsorbent in Anaerobic Digestion

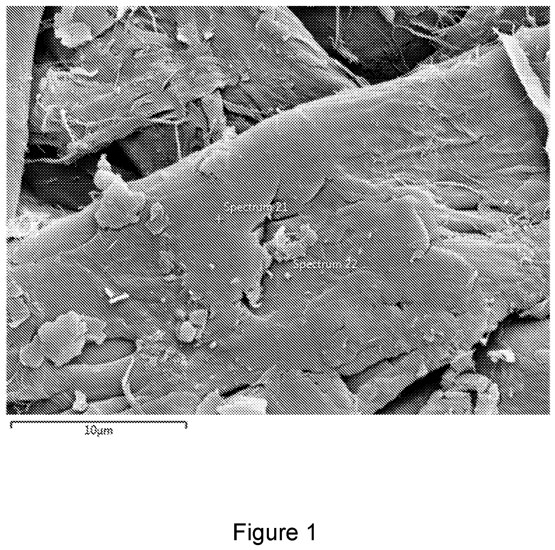

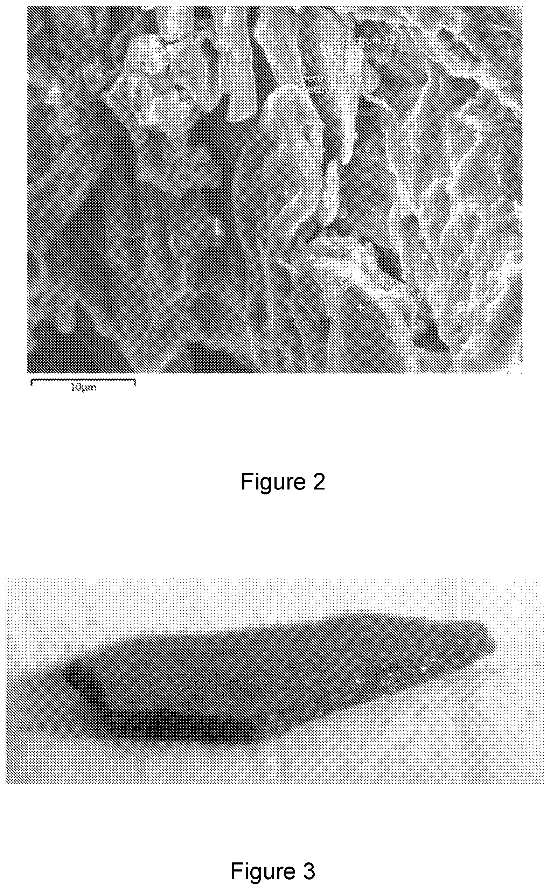

The particulate carbon adsorbent was produced from pyrolysed paper-plastic composite material. For two anaerobic digestion experiments, the paper-plastic composite material was pyrolysed into flake-shaped particles (acting as particulate carbon adsorbents according to the invention) with a carbon content of 46%, a pH of 9.7, and an electrical conductivity of 93.7 µS/cm (1:20 dilution with purified H₂O). The particles were sieved and only particles that passed through a sieve with an aperture of 0.5 mm but not through a sieve with an aperture of 0.125 mm were selected for further testing. By means of optical microscopy and a binocular magnifying glass the particulate carbon adsorbent was confirmed to have a flake shape with a vertical extent mostly between 0.02-0.30 mm. The surface of the particulate carbon adsorbent formed using the above method are shown in Figures 1 and 2. An example particulate carbon adsorbent, or "carbon flake", is shown in Figure 3, as seen via optical microscopy.

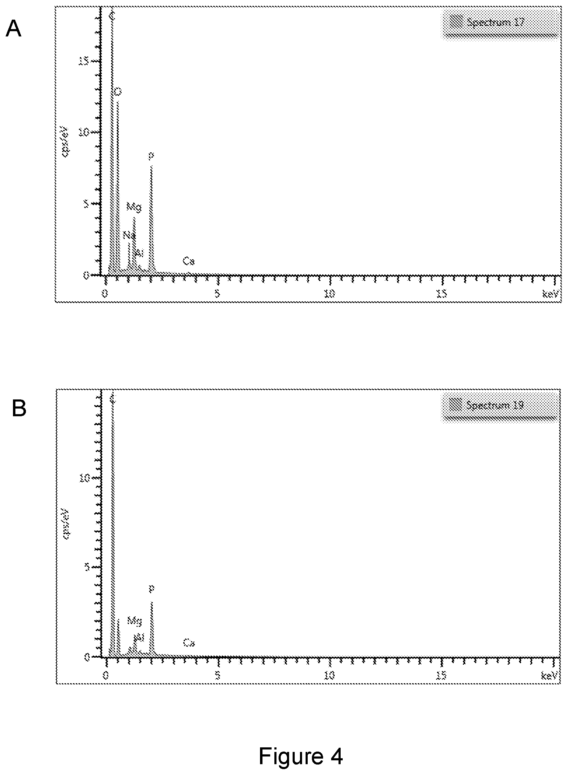

Figure 4 shows Energy-dispersive spectroscopy results from the surface of the particulate carbon adsorbent (carbon flakes) showing the elemental composition of those surfaces.

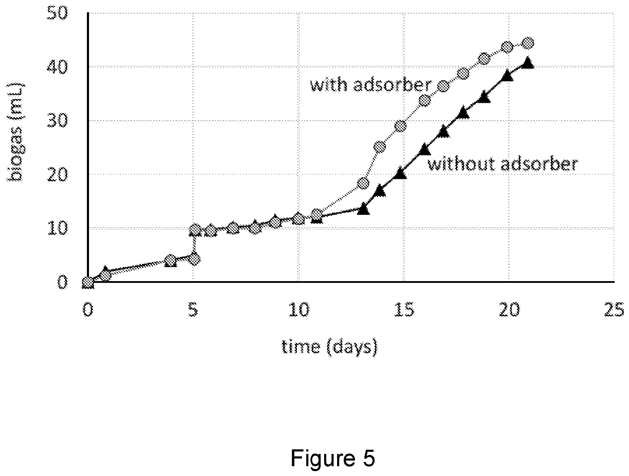

A first anaerobic digestion experiment was carried out in 60 mL plastic syringes that were continuously shaken and kept at 37°C. Initially, 8 syringes were each filled with 10 mL of inoculum from a full scale biogas plant that treats sewage sludge. In addition, 4 of the syringes were each amended with 0.2 g of the adsorbent (Carb), as particulate carbon adsorbents according to the invention. The other 4 of the syringes are referred to as the control in the following. On day 5, all syringes were fed with 1 mL of a 1% propionic acid solution to simulate inhibitory conditions. Over the total time of the experiment of 21 days, the gas production was measured frequently. The results shown in Figure 5 reveal that the biogas production in the syringes that contain the adsorbent (marked "with adsorber" in Figure 5) started earlier and was more intense than in the syringes without adsorbent (marked "without adsorber" in Figure 5).

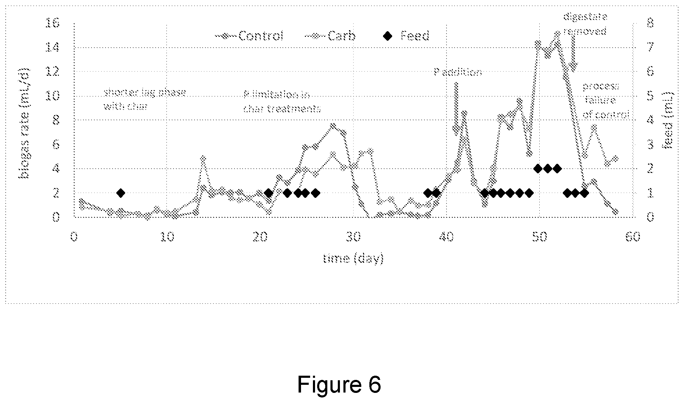

This experiment was extended under the same conditions for 58 days and the results are shown in Figure 6.

After the start-up phase of approximately two weeks the gas production of the carbon treatment declined while the control's gas rate was stable at about 2 mL per day. This was mainly attributed to having exhausted the availability of substrate. However, when feeding of propionic acid was continued on day 21, gas production of the adsorbent treatment (Carb) was relatively lower by comparison. A liquor analysis revealed very low concentrations of the phosphorus. Addition of phosphoric acid to both treatments (adsorbent (Carb) and control) on day 42 was followed by a sharp increase in biogas production. Afterwards both treatments performed similarly until digestate was removed for the first time on day 53. After about half of the syringes' content of digestate was removed the control showed an accumulation of organic acids leading to process failure, whereas the adsorbent (Carb) treatment showed stable gas production. This experiment shows that the adsorbent (Carb) material can stimulate and stabilize microbial activity. However, in order to ensure microbial activity nutrients including phosphorus are needed. Because of the adsorbent's (Carb) sorption capacity for phosphorus, the adsorbent (carb) can induce a lack of phosphorus availability in the anaerobic digestion reactor. However, this seems less likely when the adsorbent (Carb) is added in smaller instalments rather than in one batch as performed in the experiment. Alternatively, the adsorbent (carb) can be pre-charged with phosphorus before entering the anaerobic digestion reactor.

In a second anaerobic digestion experiment, the same adsorbent (Carb) was compared with other carbon materials and in addition a phosphorus-enriched variation of the adsorbent (Carb+P) was included. The phosphorus-enrichment increased the adsorbent's phosphorus content from 0.07 mg/g (Carb) to 17.8 mg/g (Carb+P). The experiment was carried out in 100 mL glass syringes that were continuously agitated in a rotating wheel and kept at 37°C. Initially, 15 syringes were each filled with 20 mL of inoculum from a full scale biogas plant that treats sewage sludge (from the same plant as the first experiment). In addition, to a set of 3 syringes 0.4 g of one each of the following carbon materials were added: Carb, Carb+P, activated carbon (AC; identical to the material used for the removal of phosphorous from an aqueous solution and for the carbon adsorbent granules), standard biochar from of the UK Biochar Research Centre made from softwood pellets at 550°C (SWP; identical to the material used for the removal of phosphorous from an aqueous solution), and the syringes with no addition of carbon material acted as Control. All materials were applied in a particle size of 0.125 to 0.5mm, except for the AC (0.063-0.25mm).

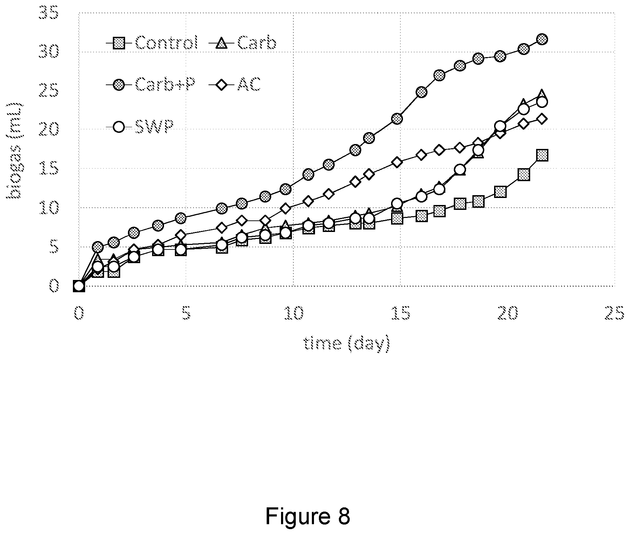

Over the total time of the experiment of 22 days, the gas production was measured frequently. The results shown in Figure 8 reveal that Carb+P had the strongest effect on biogas production. AC, Carb and SWP were also capable of increasing the biogas production but far less than Carb+P. The performance of Carb and SWP were similar and their impact on the process started later than AC. However, after 22 days, both Carb and SWP had produced more biogas than AC and the Control. This confirms the observation from the first anaerobic digestion experiment that the substantially planar carbon adsorbent (Carb) is an effective anaerobic digestion enhancer, but the microbial availability of phosphorus needs to be ensured in order to benefit from its full performance. As shown here, pre-charging or the substantially planar carbon adsorbent (Carb) with phosphorus is an effective way to overcome a potential phosphorus limitation.

In practical application, the ideal concentration of the adsorbent depends on the type and amount of inhibitory substances and the desired increase of active microbial biomass in the biogas reactor. Usually, the ideal concentration will be 0.5-3wt% of the particulate carbon adsorbent to the feedstock. Higher concentrations are not advisable because they will increase the viscosity of the biogas slurry, which makes stirring harder and could interfere with the removal of digestate. However, the ideal concentration of the adsorbent can be found and sustained by monitoring the common biogas process performance parameters e.g. methane concentration and biogas productivity and by measuring the energy consumption of the stirring devices.

### Preparation of Particulate Carbon Adsorbent with an activatable biofilm

Pre-incubation with inoculum from the target system may be carried out as follows:
1. Mix particulate carbon adsorbent with fermentation liquor from the target anaerobic digestion (AD) plant (the fermentation liquor can be obtained at any stage of the AD process; mixing can be conducted in one of the AD reactors or a separate tank, inoculum should not be stressed; particulate carbon adsorbent concentration should be between 1 and 10%)
2. Incubate for at least 24 hrs.
3. Transfer the incubated particulate carbon adsorbent to one or more reactors of the AD plant, where performance increased.

The digestate for the AD tests was obtained from the AD site at Seafield, Edinburgh UK. The plant processes thermally-hydrolysed sludge from the adjacent municipal wastewater treatment plant. AD tests were performed under the guidelines set out by the Fermentation of Organic Materials Standard VDI 4630 with the Hohenheim Biogas Yield Test batch system in 100 ml glass syringes. Reactors were supplemented with 5 g/l COD bio-oil obtained from the pyrolysis of softwood pellets at 350 degrees Celsius.

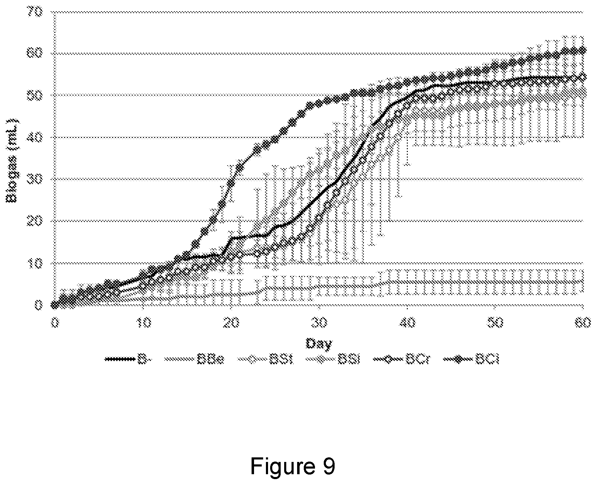

Carbon flakes were added dry or pre-incubated to the reactors to assess their impact on biogas production. Pre-incubation was achieved by adding char to the same digestate as used in the reactors to a 1:10 ratio and letting it shake at 37 Celsius for 48 hours anaerobically. Afterwards, the chars were sieved and washed with distilled water and directly added to the reactors. Reactors supplemented with pre-incubated carbon flakes showed a significantly reduced lag time before biogas production and therefore inhibition from bio-oil addition was alleviated (see Figure 9).

Without wishing to be bound by theory, it is hypothesised that pre-incubation of the biochar surfaces establishes a core microbiota that is protected from inhibitory stress due to the nature of the char surface topology and biofilm properties and thus capable of continuing AD in the presence of the stress. Conductivity of the char surface may also facilitate Direct Interspecies Electron Transfer (DIET) between microorganisms within the biofilm. The use of inoculum from the target reactor as the source of the biochar-associated microbial biofilm community ensures that no new microorganisms are being introduced to the system and thus there is no additional competition for niches within the reactor environment.

### Use of Carbon Adsorbent for removal of phosphorous (P) from an aqueous solution

For a phosphorous adsorption experiment following carbon adsorbent materials were tested:
1. PE paper cup #1 (PC1) pyrolysed at 450°C with particle size of 0.125-0.5mm
2. PE paper cup #1 (PC1) pyrolysed at 750°C with particle size of 0.125-0.5mm
3. PE paper cup #2 (PC2) pyrolysed at 450°C with particle size of 0.125-0.5mm
4. PE paper cup #2 (PC2) pyrolysed at 550°C with particle size of 0.125-0.5mm
5. PE paper cup #2 (PC2) pyrolysed at 750°C with particle size of 0.125-0.5mm
6. PE paper cup #2 (PC2) pyrolysed at 450°C with particle size of <0.125mm
7. PE paper cup #2 (PC2) pyrolysed at 750°C with particle size of 0.063-0.125mm
8. PLA paper cup (PC3) pyrolysed at 450°C with particle size of 0.125-0.5mm
9. PLA paper cup (PC3) pyrolysed at 750°C with particle size of 0.125-0.5mm
10. Activated carbon (AC) with particle size of 0.063-0.25mm
11. Standard biochar of the UK Biochar Research Centre made from softwood pellets at 550°C (SWP) with particle size of 0.125-0.5mm
12. Control without adsorbent

PC1 is an 8oz white single walled polyethylene (PE)-laminated paper cup made in China for Sainsbury's.

PC2 is a 12oz black doubled walled PE-laminated paper cup branded for Marks & Spencer and made by Euro Packaging UK Ltd.

PC3 is an 8oz brown single walled polylactic acid (PLA)-laminated paper cup made by Vegware Ltd.

Materials 2, 5, 7, 9 and 12 were tested in triplicate, the others as single experiments.

A phosphorous solution was prepared using K₂HPO₄ and deionized water with a measured phosphorous concentration to achieve a phosphorous concentration of 20 mgL⁻¹, which is a relevant concentration in waste waters. The sorption experiment was carried out in centrifuge tubes filled with 10 mg of carbon material and 20 g of the phosphorous solution. The tubes were kept on a shaker. After 24 hrs, the liquid phase was analysed for its phosphorous content and the specific amount of adsorbed (or desorbed) phosphorous by the carbon material was calculated using the control as basis.

The highest phosphorous sorption was found for the carbon adsorbent that was produced at 750°C (see Table B). This can be attributed be to a higher concentration of Mg and Ca and in particular to a higher concentration of MgO at higher pyrolysis temperatures as shown in Tab. 1.

**Table 2: Measured amount of adsorbed phosphorous by various carbon materials after 24h (NA = not available)**

| Carbon material | Pyrolysis temperatur e | Particle size range | Adsorbed phosphorous by carbon material after 24h |
|---|---|---|---|
| | °C | mm | mg P/g |
| PE paper cup #1 (PC1) | 750 | 0.125-0.5 | 18.05 |
| | 450 | 0.125-0.5 | -0.34 |
| PE paper cup #2 (PC2) | 750 | 0.125-0.5 | 4.93 |
| | 750 | 0.063-0.125 | 23.01 |
| | 550 | 0.125-0.5 | 2.24 |
| | 450 | 0.125-0.5 | 0.16 |
| | 450 | <0.125 | -0.04 |
| PLE paper cup (PC3) | 750 | 0.125-0.5 | 22.65 |
| | 450 | 0.125-0.5 | -0.21 |
| AC | NA | 0.063-0.25 | -16.40 |
| WP | 550 | 0.125-0.5 | -0.10 |

| | | | |
|---|---|---|---|
| AC showed a negative value of -16.4 mg g⁻¹, which indicates that the AC was activated using phosphoric acid that now leached into the solution. | | | |

All materials that were produced at 450°C and SWP were in the range of -0.35 to 0.16 mg g⁻¹. The carbon adsorbent produced at 550°C from PC2 was found to have an adsorption performance in between the 450°C and 750°C adsorbents. This corresponds to the formation of MgO starting at 550°C as shown in Table 1. The adsorption experiment with this material was continued for a retention time of 5 days, which increased the measured adsorption to an average of 5.6 mg g⁻¹. This shows that a longer retention time can increase the level of phosphorous adsorption. Further, it was also shown that the performance of the 750°C adsorbents are affected by particle size as can be seen when comparing the results from the PC2 materials in both tested particle sizes. The higher sorption capacity of smaller particles can be explained by a higher surface area and more effective Mg and Ca bonding sites.

Accordingly, these experiments show that the particulate carbon adsorbents of the invention are effective phosphorous adsorbents. In order to be effective the particulate carbon adsorbent should be produced at temperatures of at least 550°C and from feedstock materials that have a Mg content of at least 1wt%. The sorption capacity can be increased by grinding the adsorbent into smaller particles sizes. However, smaller particles are more difficult to separate from the treated wastewater and Mg and Ca compounds are more likely to become detached from the carbon adsorbent. Thus, in most applications a particle size in the range of 0.05mm to 5mm is ideal.

### Carbon granules

In an embodiment, the carbon flakes for the carbon granule's shell are produced from pyrolysed paper-plastic composite material and a seaweed extract is used as binder for both the core and shell. For an agglomeration experiment, the paper-plastic composite material was pyrolysed into flake-shaped particles with a carbon content of 46%, a pH of 9.7, and an electrical conductivity of 93,7 µS/cm (1:20 dilution with purified H₂O). The particles were sieved and only particles that passed through a sieve with an aperture of 0.5 mm but not through a sieve with an aperture of 0.125mm were selected for further testing. By means of optical microscopy and a binocular magnifying glass the carbon particles were confirmed to have a flake-shape with a vertical extent mostly between 0.02mm to 0.30mm. A commercial seaweed-extract (Original Organic Seaweed Extract by MaxiCrop) with a total solids content of 8% and a pH of 8.5 was used as binder. As core material for the agglomerates, activated carbon with a mesh size of 100 to 400 was purchased from Sigma Aldrich. The agglomeration was performed in a wet drum granulator using a round tin box with a diameter of 24cm and length of 13cm. The initial amounts of activated carbon used for granulation were 17.4 g (for a control without a shell), 20.6 g for a granule with an intended mass-based core-to-shell ratio of 20:1 (thin shell) and 10.7 g for a granule with a mass-based core-to-shell ratio of 1:1 (thick shell). The right amount of activated carbon was fed into the drum and granulated at 60 rpm. Each three minutes the granulation process was checked. The binder, that was diluted 1:1 with H₂O, was added in 4-6 instalments by means of a spray bottle. The total amount of binder corresponded to a binder-to-activated carbon ratio of 1.8 to 1.

When granulation of the activated carbon was visible after 21 minutes (thin shell granule) or 15 minutes (thick shell granule), the right amount of carbon flakes was added into the drum. For the thin-shelled granule, granulation was continued for 10 min and no further binder was added. For the thick-shelled granule, granulation was continued for 21 minutes with 4 instalments of binder addition. In total, 1.2 mass parts of binder were added to 1 mass part of carbon flakes. The control batch without a shell was granulated for a total of 20 min. After granulation, the granular agglomerates were dried at 105°C until a stable weight was observed. The granule diameter varied mostly between 1-10 mm.

For testing the mechanical stability only granules with a diameter of 5-8 mm were selected. To simulate harsh mechanical stress, the granules were subjected to 5 and 10 min inside the same drum as used for granulation run at 60 rpm. After 5 and 10 min, 21% and 58% of the granules without shells were crushed, whereas the thin-shelled granules crushed by only 8 and 22% and the thick-shelled granules by only 3% and 3%. This shows that carbon agglomerates with a shell of flake-shaped carbon particles have a significant higher durability than unshelled agglomerates. Further, the produced carbon granules were tested for their disintegration behaviour in water and all granule types were observed to disintegrate instantly as desired.

In another embodiment, no binder but only water is used to agglomerate the carbon particles for the core granule. Binder was only used to form the shell of carbon flakes. Again, the carbon flakes for the granule's shell are produced from pyrolysed paper-plastic composite material and a seaweed extract is used as binder.

For an agglomeration experiment, the same activated carbon, carbon flakes, binder and wet drum granulator were used as in the previous experiment. The amount of activated carbon was 16.2 g. Before granulation, 16.2 g of water was added to the activated carbon. The material was granulated for 5 minutes then further 5.3 g of H₂O was added. After additional 10 minutes, formation of granules with diameters of 5-15 mm were observed and 1.62 of carbon flakes for shell formation were added. After further 8 minutes of granulation, most of the carbon flakes were picked up by the wet granules to form the shell as desired. Then 1.62 g of the seaweed extract (no dilution) was sprayed on the granules and granulation was continued for further 3 minutes. Subsequently, a second layer of carbon flakes was added to the granules by adding further 1.62 g of carbon flakes and 1.62 g of seaweed extract. Finally, after further granulation for 7 minutes, a third batch of 1.62 g of seaweed extract was sprayed on the granules and granulation was continued for further 7 minutes. After this, granulation was stopped and the granules were dried at 60°C until their weight was stable. The granules were observed to have a 1 mm larger diameter due to the shell. In contrast to agglomerated activated carbon that was produced with neither shell nor binder and that were observed to already disintegrate during drying, the shelled carbon granules sustained the drying process fully intact. When some granules were cut open their content of activated carbon was found in loose state. Thus no significant amounts of binder had entered the granules' core zone.

For practical application, all types of wet drum granulates can be used to produce the shelled-carbon granules. However, preferably the system allows continuous spray feeding of the binder as well as continuous feeding of carbon particles. Drying could be performed in the same granulator or separately by various batch or continuous types of dryers e.g. belt dryers. After the drying step, additional treatments can be conducted in order to remove dust and granules that are outside the desired size range. This can be achieved by common mechanical or pneumatic separation systems. In cases where the binder and flake-shaped carbon particles are tolerated inside the granules' core, removed dust and outsized granules can be recycled to the agglomeration process. Therefore, the outsized granules should be crushed first. In a preferred setup, the heat for drying the granules is obtained from an onsite pyrolysis kiln that produces the carbon particles for either the granules' core, the shell or both. The pyrolysis gas that is produced during pyrolysis can be burned onsite to fuel the drying process.

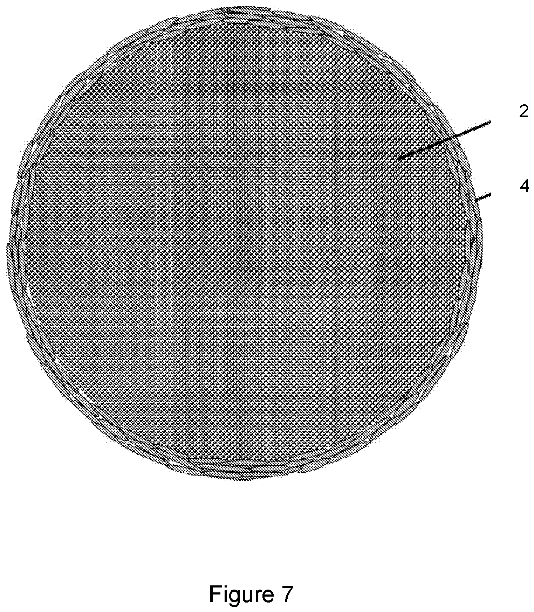

Figure 7 shows an embodiment of the granular aggregate comprising a core of smaller carbon particles (1) and a shell of larger flake-shaped particles (2).

The carbon adsorbent granules so produced can then be used in a number of applications, such as in anaerobic digestion, treatment of wastewater, phosphorous capture and soil amendment.

The following methods and apparatus were used in order to determine the parameters given with regard to the invention:

### Atomic adsorption (for total Mg and Ca analysis of the particulate carbon adsorbent)

The solid samples were digested as described in the following: 0.5 g of sample material was weighed into crucibles, heated to 500°C and held at this temperature for 8 h. After cooling, the samples were placed in a steam bath and 5 mL of concentrated (70%) HNO₃ (analytical grade, Fisher Scientific) was added and evaporated to dryness. After cooling, 1 mL HNO₃ and 4 mL H₂O₂ (30%, analytical grade, Fisher Scientific) were added and evaporated to dryness. Next, 2 mL HNO₃ was added to dissolve the solids. The resulting solution was filtered through Whatman No. 41 filter paper and the volume increased to 50 mL with DI water. The solutions were than analysed by a ThermoFisher Scientific ICE 3000 atomic absorption spectrometer.

### Phosphorous analysis (for the particulate carbon adsorbent and phosphorus solution)

For solid samples, the some digestion as for atomic adsorption was used. The solutions were analysed by automated colorimetry (Auto Analyser III, Bran & Luebbe, Norderstedt, Germany).

### XRD (for identification of Mg bonding types in the carbon adsorbent)

A Bruker AXS D8 Advance Diffractometer with copper anode x-ray tube and Sol-X Energy Dispersive detector was used. The running conditions for the X-Ray tube were 40 kV and 40 mA. Experiments were carried out in the range of 2° ≤ 2Θ ≤ 65° with a step size of 0.05° and 1 sec per step. Data analysis was performed by Bruker's DIFFRAC EVA software and Bruker's TOPAS software was used for semiquantitative analysis. The samples were analysed in powdery form with a particle size below 0.125mm.

### EDS (for determination of the mineral composition on the particulate carbon adsorbent's surface

A Carl Zeiss SIGMA HD VP Field Emission SEM and Oxford AZtec ED X-ray analysis were used.

### Elemental analysis (for the carbon content of the particulate carbon adsorbent)

The elemental (CHN) analysis was performed in quadruplicates using a Flash 2000 Elemental Analyser.

### References

Chen et al. 2011, Bioresour Technol 102, 716-723.
Park et al. 2015, Env Geochem Health 37, 969-983
Ren et al. 2015, Bioresource Technology 178, 119-125
Yao et al. 2011, Bioresour Technol 102, 6273-6278.
Yao et al., 2013a, Bioresource Technology 138, 8-13
Zhang et al. 2012, Chem Eng J 210, 26-32.
Zhang et al. 2013, Chemosphere 92, 1042-1047
Zheng et al. 2010, Using Biochar as a Soil Amendment for Sustainable Agriculture, Project Report, University of Illinois at Urbana-Champaign

## Claims

1. A particulate carbon adsorbent for use in anaerobic digestion, the particulate carbon adsorbent being substantially planar and comprising between 40-90 wt% carbon, **characterised in** the particulate carbon adsorbent is obtainable by pyrolysing a substantially planar organic material at a temperature from 300°C to 800°C, grinding the pyrolysed material; and sorting the ground pyrolysed material to specify the dimensions of the particulate carbon adsorbent.

2. The particulate of claim 1, wherein the temperature for pyrolysing the substantially planar organic material is from 400°C to about 475°C, or wherein the temperature for pyrolysing the substantially planar organic material is from 650°C to about 800°C.

3. The particulate carbon adsorbent for use in anaerobic digestion according to claim 1,wherein the anaerobic digestion is carried out in a bioreactor, and as a result, the particulate carbon adsorbent is configured to be used in a bioreactor.

4. The particulate carbon adsorbent for use in anaerobic digestion according to any preceding claim, wherein the particulate carbon adsorbent is a substrate for the formation of an active biofilm.

5. The particulate carbon adsorbent for use in anaerobic digestion according to any preceding claim, wherein the particulate carbon adsorbent comprises an active biofilm, optionally prior to use.

6. The particulate carbon adsorbent for use in anaerobic digestion according to claim 5, wherein the particulate carbon adsorbent comprises microorganisms pre-inoculated from within fluid obtained from a target system at any stage of the anaerobic digestion process.

7. The particulate carbon adsorbent for use in anaerobic digestion according to any preceding claim, wherein the particulate carbon adsorbent comprises calcium and/or magnesium ions.

8. The particulate carbon adsorbent for use in anaerobic digestion according to claim 7,
wherein the particulate carbon adsorbent comprises from about 0.1 wt % to about 10 wt% calcium ions; and/or
wherein the particulate carbon adsorbent comprises from about 1.0 wt% to about 15.0 wt% magnesium ions.

9. A method of manufacture of particulate carbon adsorbents for the use in anaerobic digestion, the particulate carbon adsorbent being substantially planar and comprising between 40-90 wt% carbon, the method comprising the steps:
a) providing a substantially planar organic material;
b) pyrolysing the provided substantially planar organic material at a temperature from 300°C to 800°C;
c) grinding the pyrolysed material resulting from step b); and
d) sorting the ground pyrolysed material resulting from step c) to specify the dimensions of the particulate carbon adsorbent required.

10. The method of claim 9, wherein the substantially planar organic feedstock is pyrolysed at a temperature from 400°C to about 475°C, or wherein the substantially planar organic feedstock is pyrolysed at a temperature from 650°C to about 800°C.

11. The method of any of claim 9 or claim 10, wherein the method further comprises the step of shredding the substantially planar organic material before it is pyrolysed.

12. The method of claim 11, wherein the method further comprises the step of compressing the shredded substantially planar organic material before the step of pyrolysing the substantially planar organic material.

13. The method of any of claim 9 to claim 12, wherein the substantially planar organic feedstock comprises paper, cardboard, or a composite material formed from layers of paper, layers of plastic, and/or wax and/or aluminium.

14. An anaerobic digestion process comprising the steps:
a) providing a bioreactor;
b) adding a feedstock to the bioreactor;
c) adding a microorganism composition to the bioreactor;
d) adding an adsorbent composition to the bioreactor, the adsorbent composition comprising substantially planar particulate carbon adsorbent as defined in any one of claims 1 to 8;
e) incubating the bioreactor such that microorganisms within the microorganism composition digest the feedstock to produce a digestate and biogas; and
f) removing the produced biogas and digestate.

15. The method of claim 14, wherein the adsorbent composition is pre-inoculated prior to addition to the bioreactor with fluid from the bioreactor that contains the feedstock and microorganism composition of that bioreactor.

## Patentansprüche

1. Ein teilchenförmiges Kohlenstoffadsorptionsmittel zur Verwendung bei der anaeroben Vergärung, wobei das teilchenförmige Kohlenstoffadsorptionsmittel im Wesentlichen planar ist und zu zwischen 40 und 90 Gew.-% Kohlenstoff beinhaltet, **dadurch gekennzeichnet, dass** das teilchenförmige Kohlenstoffadsorptionsmittel durch Pyrolysieren eines im Wesentlichen planaren organischen Materials bei einer Temperatur von 300 °C bis 800 °C, Mahlen des pyrolysierten Materials und Sortieren des gemahlenen pyrolysierten Materials, um die Abmessungen des teilchenförmigen Kohlenstoffadsorptionsmittels zu spezifizieren, erhalten werden kann.

2. Teilchenförmiges Mittel gemäß Anspruch 1, wobei die Temperatur zum Pyrolysieren des im Wesentlichen planaren organischen Materials von 400 °C bis etwa 475 °C beträgt oder wobei die Temperatur zum Pyrolysieren des im Wesentlichen planaren organischen Materials von 650 °C bis etwa 800 °C beträgt.

3. Teilchenförmiges Kohlenstoffadsorptionsmittel zur Verwendung bei der anaeroben Vergärung gemäß Anspruch 1, wobei die anaerobe Vergärung in einem Bioreaktor ausgeführt wird und das teilchenförmige Kohlenstoffadsorptionsmittel folglich zur Verwendung in einem Bioreaktor ausgelegt ist.

4. Teilchenförmiges Kohlenstoffadsorptionsmittel zur Verwendung bei der anaeroben Vergärung gemäß einem der vorhergehenden Ansprüche, wobei das teilchenförmige Kohlenstoffadsorptionsmittel ein Substrat zur Bildung eines aktiven Biofilms ist.

5. Teilchenförmiges Kohlenstoffadsorptionsmittel zur Verwendung bei der anaeroben Vergärung gemäß einem der vorhergehenden Ansprüche, wobei das teilchenförmige Kohlenstoffadsorptionsmittel einen aktiven Biofilm, optional vor der Verwendung, beinhaltet.

6. Teilchenförmiges Kohlenstoffadsorptionsmittel zur Verwendung bei der anaeroben Vergärung gemäß Anspruch 5, wobei das teilchenförmige Kohlenstoffadsorptionsmittel Mikroorganismen beinhaltet, die von innerhalb aus einem Zielsystem in einem beliebigen Stadium des anaeroben Vergärungsprozesses erhaltenen Fluid vorgeimpft wurden.

7. Teilchenförmiges Kohlenstoffadsorptionsmittel zur Verwendung bei der anaeroben Vergärung gemäß einem der vorhergehenden Ansprüche, wobei das teilchenförmige Kohlenstoffadsorptionsmittel Calcium- und/oder Magnesiumionen beinhaltet.

8. Teilchenförmiges Kohlenstoffadsorptionsmittel zur Verwendung bei der anaeroben Vergärung gemäß Anspruch 7, wobei das teilchenförmige Kohlenstoffadsorptionsmittel zu etwa 0,1 Gew.-% bis etwa 10 Gew.-% Calciumionen beinhaltet; und/oder wobei das teilchenförmige Kohlenstoffadsorptionsmittel zu etwa 1,0 Gew.-% bis etwa 15,0 Gew.-% Magnesiumionen beinhaltet.

9. Ein Verfahren zur Herstellung teilchenförmiger Kohlenstoffadsorptionsmittel zur Verwendung bei der anaeroben Vergärung, wobei das teilchenförmige Kohlenstoffadsorptionsmittel im Wesentlichen planar ist und zu zwischen 40 und 90 Gew.-% Kohlenstoff beinhaltet, wobei das Verfahren die folgenden Schritte beinhaltet:
a) Bereitstellen eines im Wesentlichen planaren organischen Materials;
b) Pyrolysieren des bereitgestellten im Wesentlichen planaren organischen Materials bei einer Temperatur von 300 °C bis 800 °C;
c) Mahlen des aus Schritt b) resultierenden pyrolysierten Materials; und
d) Sortieren des aus Schritt c) resultierenden gemahlenen pyrolysierten Materials, um die benötigten Abmessungen des teilchenförmigen Kohlenstoffadsorptionsmittels zu spezifizieren.

10. Verfahren gemäß Anspruch 9, wobei das im Wesentlichen planare organische Beschickungsmaterial bei einer Temperatur von 400 °C bis etwa 475 °C pyrolysiert wird oder wobei das im Wesentlichen planare organische Beschickungsmaterial bei einer Temperatur von 650 °C bis etwa 800 °C pyrolysiert wird.

11. Verfahren gemäß Anspruch 9 oder Anspruch 10, wobei das Verfahren ferner den Schritt des Zerkleinerns des im Wesentlichen planaren organischen Materials, bevor es pyrolysiert wird, beinhaltet.

12. Verfahren gemäß Anspruch 11, wobei das Verfahren vor dem Schritt des Pyrolysierens des im Wesentlichen planaren organischen Materials ferner den Schritt des Komprimierens des zerkleinerten im Wesentlichen planaren organischen Materials beinhaltet.

13. Verfahren gemäß Anspruch 9 bis Anspruch 12, wobei das im Wesentlichen planare organische Beschickungsmaterial Papier, Pappe oder ein Verbundstoffmaterial, das aus Schichten von Papier, Schichten von Kunststoff und/oder Wachs und/oder Aluminium gebildet ist, beinhaltet.

14. Ein anaerober Vergärungsprozess, der die folgenden Schritte beinhaltet:
a) Bereitstellen eines Bioreaktors;
b) Hinzufügen eines Beschickungsmaterials zu dem Bioreaktor;
c) Hinzufügen einer Mikroorganismenzusammensetzung zu dem Bioreaktor;
d) Hinzufügen einer Adsorptionsmittelzusammensetzung zu dem Bioreaktor, wobei die Adsorptionsmittelzusammensetzung im Wesentlichen planares teilchenförmiges Kohlenstoffadsorptionsmittel gemäß einem der Ansprüche 1 bis 8 beinhaltet;
e) Inkubieren des Bioreaktors, sodass die Mikroorganismen in der Mikroorganismenzusammensetzung das Beschickungsmaterial vergären, um einen Gärrest und Biogas zu produzieren; und
f) Entfernen des produzierten Biogases und Gärrests.

15. Verfahren gemäß Anspruch 14, wobei die Adsorptionsmittelzusammensetzung vor dem Hinzufügen zu dem Bioreaktor mit Fluid von dem Bioreaktor, das das Beschickungsmaterial und die Mikroorganismenzusammensetzung dieses Bioreaktors enthält, vorgeimpft wird.

## Revendications

1. Un adsorbant en carbone particulaire pour une utilisation dans une digestion anaérobie, l'adsorbant en carbone particulaire étant sensiblement plan et comprenant entre 40 et 90 % en poids de carbone, **caractérisé en ce que** l'adsorbant en carbone particulaire peut être obtenu par pyrolyse d'un matériau organique sensiblement plan à une température allant de 300 °C à 800 °C, broyage du matériau pyrolysé ; et tri du matériau pyrolysé broyé afin de spécifier les dimensions de l'adsorbant en carbone particulaire.

2. La matière particulaire de la revendication 1, dans laquelle la température pour la pyrolyse du matériau organique sensiblement plan va de 400 °C à environ 475 °C, ou dans laquelle la température pour la pyrolyse du matériau organique sensiblement plan va de 650 °C à environ 800 °C.

3. L'adsorbant en carbone particulaire pour une utilisation dans une digestion anaérobie selon la revendication 1, dans lequel la digestion anaérobie est réalisée dans un bioréacteur, et par conséquent, l'adsorbant en carbone particulaire est configuré pour être utilisé dans un bioréacteur.

4. L'adsorbant en carbone particulaire pour une utilisation dans une digestion anaérobie selon n'importe quelle revendication précédente, l'adsorbant en carbone particulaire étant un substrat pour la formation d'un biofilm actif.

5. L'adsorbant en carbone particulaire pour une utilisation dans une digestion anaérobie selon n'importe quelle revendication précédente, l'adsorbant en carbone particulaire comprenant un biofilm actif, facultativement avant l'utilisation.

6. L'adsorbant en carbone particulaire pour une utilisation dans une digestion anaérobie selon la revendication 5, l'adsorbant en carbone particulaire comprenant des microorganismes pré-inoculés à partir d'un fluide obtenu à partir d'un système cible à n'importe quelle étape du processus de digestion anaérobie.

7. L'adsorbant en carbone particulaire pour une utilisation dans une digestion anaérobie selon n'importe quelle revendication précédente, l'adsorbant en carbone particulaire comprenant des ions calcium et/ou magnésium

8. L'adsorbant en carbone particulaire pour une utilisation dans une digestion anaérobie selon la revendication 7, l'adsorbant en carbone particulaire comprenant d'environ 0,1 % en poids à environ 10 % en poids d'ions calcium ; et/ou l'adsorbant en carbone particulaire comprenant d'environ 1,0 % en poids à environ 15,0 % en poids d'ions magnésium.

9. Un procédé de fabrication d'adsorbants en carbone particulaires pour l'utilisation dans une digestion anaérobie, l'adsorbant en carbone particulaire étant sensiblement plan et comprenant entre 40 et 90 % en poids de carbone, le procédé comprenant les étapes :
a) de mise à disposition d'un matériau organique sensiblement plan ;
b) de pyrolyse du matériau organique sensiblement plan mis à disposition à une température allant de 300 °C à 800 °C ;
c) de broyage du matériau pyrolysé issu de l'étape b) ; et
d) de tri du matériau pyrolysé broyé issu de l'étape c) afin de spécifier les dimensions de l'adsorbant en carbone particulaire requis.

10. Le procédé de la revendication 9, dans lequel le produit de départ organique sensiblement plan est pyrolysé à une température allant de 400 °C à environ 475 °C, ou dans lequel le produit de départ organique sensiblement plan est pyrolysé à une température allant de 650 °C à environ 800 °C

11. Le procédé de n'importe quelle revendication de la revendication 9 ou de la revendication 10, le procédé comprenant en outre l'étape de déchiquetage du matériau organique sensiblement plan avant qu'il ne soit pyrolysé.

12. Le procédé de la revendication 11, le procédé comprenant en outre l'étape de compression du matériau organique sensiblement plan déchiqueté avant l'étape de pyrolyse du matériau organique sensiblement plan.

13. Le procédé de n'importe quelle revendication de la revendication 9 à la revendication 12, dans lequel le produit de départ organique sensiblement plan comprend du papier, du carton, ou un matériau composite formé à partir de couches de papier, de couches de plastique, et/ou de cire et/ou d'aluminium.

14. Un processus de digestion anaérobie comprenant les étapes :
a) de mise à disposition d'un bioréacteur ;
b) d'ajout d'un produit de départ dans le bioréacteur ;
c) d'ajout d'une composition de microorganismes dans le bioréacteur ;
d) d'ajout d'une composition d'adsorbant dans le bioréacteur, la composition d'adsorbant comprenant l'adsorbant en carbone particulaire sensiblement plan tel que défini dans n'importe laquelle des revendications 1 à 8 ;
e) d'incubation du bioréacteur de telle sorte que des microorganismes au sein de la composition de microorganismes digèrent le produit de départ afin de produire un digestat et un biogaz ; et
f) de retrait du biogaz et du digestat produits.

15. Le procédé de la revendication 14, dans lequel la composition d'adsorbant est pré-inoculée avant l'ajout dans le bioréacteur avec du fluide du bioréacteur qui contient le produit de départ et la composition de microorganismes de ce bioréacteur.
